(19) **Europäisches Patentamt**
**European Patent Office**
**Office européen des brevets**

(11) **EP 4 429 562 B1**

(12) **EUROPEAN PATENT SPECIFICATION**

(45) Date of publication and mention
of the grant of the patent:
**06.11.2024 Bulletin 2024/45**

(21) Application number: **23814462.0**

(22) Date of filing: **30.11.2023**

(51) International Patent Classification (IPC):
***A61B 8/08*** *(2006.01)* ***A61B 8/06*** *(2006.01)*
***A61B 8/12*** *(2006.01)*

(52) Cooperative Patent Classification (CPC):
**A61B 8/06; A61B 8/12; A61B 8/488; A61B 8/5223;**
**A61B 8/5246**

(86) International application number:
**PCT/EP2023/083692**

(87) International publication number:
**WO 2024/115639 (06.06.2024 Gazette 2024/23)**

(54) **DETERMINING A FLOW PROFILE IN AN ARTERY BASED ON ULTRASOUND IMAGING DATA**

BESTIMMUNG EINES STRÖMUNGSPROFILS IN EINER ARTERIE BASIEREND AUF
ULTRASCHALLBILDGEBUNGSDATEN

DÉTERMINATION D'UN PROFIL D'ÉCOULEMENT DANS UNE ARTÈRE SUR LA BASE DE
DONNÉES D'IMAGERIE ULTRASONORE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **02.12.2022 EP 22210995**
**08.12.2022 EP 22212312**

(43) Date of publication of application:
**18.09.2024 Bulletin 2024/38**

(73) Proprietor: **Koninklijke Philips N.V.**
**5656 AG Eindhoven (NL)**

(72) Inventors:
• **SHULEPOV, Sergey Yuryevich**
**5656 AG Eindhoven (NL)**
• **JOSHI, Rohan**
**5656 AG Eindhoven (NL)**

• **VAN ROOIJ, Johannes Antonius**
**5656 AG Eindhoven (NL)**

(74) Representative: **Philips Intellectual Property &**
**Standards**
**High Tech Campus 52**
**5656 AG Eindhoven (NL)**

(56) References cited:
**WO-A2-2021/250234 US-A1- 2012 078 106**

• **TIMOTHY IRVINE ET AL: "A digital 3-dimensional**
**method for computing great artery flows: In vitro**
**validation studies", JOURNAL OF THE**
**AMERICAN SOCIETY OF**
**ECHOCARDIOGRAPHY., vol. 13, no. 9, 1**
**September 2000 (2000-09-01), US, pages 841 -**
**848, XP055705599, ISSN: 0894-7317, DOI:**
**10.1067/mje.2000.106049**

**Description**

FIELD OF THE INVENTION

The invention relates to determining a flow profile in an artery based on ultrasound imaging data.

BACKGROUND OF THE INVENTION

When determining blood flow in an artery using ultrasound, a sonographer typically positions an ultrasound probe with the imaging plane in the middle of the artery and along the main axis of the flow. During the examination, the sonographer then typically utilizes two types of measurements: a color or power Doppler measurements to assess the flow distribution over the lumen of the artery, and a pulsed wave Doppler measurement to obtain the blood velocity waveform. An example method for continuous non-invasive monitoring of multiple arterial parameters of a patient is provided in US 2012/0078106. This procedure, however, assumes that the velocity distribution is axially symmetric, and the maximum velocity is close to the artery or lumen center. However, most of the arteries are not straight, and tortuosity of the arteries also increases with age of the patient.

SUMMARY OF THE INVENTION

It is, inter alia, an object of the invention to determine a flow profile in an artery with increased accuracy, and in particular in arteries with increased tortuosity.
The invention is defined by the independent claims. Advantageous embodiments are defined in the dependent claims.
According to an aspect of the invention, there is provided a computer-implemented method for determining a flow profile in an artery based on ultrasound imaging data, the method comprising:

- receiving ultrasound imaging data of an artery in triplex mode, wherein the triplex mode comprises:

    - B-mode ultrasound data,
    - pulsed wave Doppler ultrasound data, and
    - color or power Doppler ultrasound data;

- determining (320), from the ultrasound imaging data,

    - an artery dimension;
    - a velocity in the artery cross-section, and
    - a measure of asymmetry of the flow profile; and

- determining a flow profile from

    - the artery dimension,
    - the velocity in the artery cross-section, and
    - the measure of asymmetry of the flow profile.

In this manner, by obtaining a few measurement points from ultrasound imaging data (i.e., an artery dimension, a velocity in the artery cross-section, and a measure of asymmetry of the flow), an accurate flow profile can be determined according to analytical expressions. In other words, the flow profile is re-constructed with the knowledge of parabolic flows and the parameters determined from the ultrasound imaging data. The method may further be carried out during or after an ultrasound imaging procedure.
The method of may further comprise:

- determining a local artery curvature based on

    - the artery dimension,
    - the flow profile, and
    - the measure of asymmetry of the flow profile.

By determining a local artery curvature, it is possible to improve for example methods for plaque removal and/or stent placements.

In an example the artery dimension is determined based on the B-mode ultrasound data. In an example the velocity in the artery cross-section is determined based on the pulsed wave Doppler ultrasound data. In an example, the velocity in the artery cross-section is determined in a center of the artery cross-section. In an example, the measure of asymmetry of the flow profile is determined based on the color or power Doppler ultrasound data.

In an example, the measure of asymmetry comprisesdetermining a location of the maximum flow velocity in an artery cross-section; and based on said location, determining a parameter representative of a deviation of said location with respect to the center of the artery cross-section.

In an example, the parameter representative of the deviation of the location of the maximum flow velocity in an artery cross-section is the Dean number.

According to an aspect of the invention, there is provided a computer program product comprising instructions for enabling a processor to carry out the above method. The computer program product may be software available for download from a server, e.g., via the internet. Alternatively, the computer program product may be a suitable (non-transitory) computer readable medium on which the instructions are stored, such as an optical storage medium or a solid-state medium supplied together with or as part of other hardware.

According to an aspect of the invention, there is provided a system for determining a flow profile from ultrasound imaging data, the system comprising a processor configured to carry out the above method.

In an example, the system may further comprise

- an array of acoustic elements in communication with the processor, and
- wherein the processor is configured to control the array of acoustic elements to send and receive acoustic signals to generate ultrasound imaging data.

In an example, the system is a hemodynamic monitoring patch.

These and other aspects of the invention will be apparent from and elucidated with reference to the embodiments described hereinafter.

Definitions

"cross-section" refers to an intersection of an artery in three-dimensional space with an imaging plane. A "cross-section" of the artery is thus any plane intersecting the artery itself, not necessarily at a 90-degree angle.

"Transversal cross-section" is used to refer to a plane intersecting the artery, wherein said plane forms a 90 degree angle with the length of the artery. A cross-section of the artery thus includes the possibility of a transversal cross-section.

"maximum velocity" is used to refer to the maximum velocity encountered in a velocity profile in a transversal cross-section of an artery.

"peak velocity" is used to refer to the peak velocity in a transient (i.e., evolving in time) velocity profile at a location. For example, a peak in a transient velocity profile from pulsed wave Doppler data.

"center-line" refers to the symmetry axis along the length of the artery. In other words, the axis of the cylinder that represents an artery.

"shift in maximum velocity" or "maximum velocity shift" or "velocity maximum shift" or any other combination of these words, refers to the shift of the maximum velocity in a velocity profile with respect to the centroid of the artery cross-section. In other words, the distance between the location of the velocity maximum and the central axes of the artery.

BRIEF DESCRIPTION OF THE DRAWINGS

Fig. 1 shows a schematic of an ultrasound system in which the invention may be used.
Fig. 2 shows a schematic of a processing circuit that may be used in an embodiment.
Fig. 3 shows a diagram of a method according to an embodiment.
Fig. 4 shows an exemplary B-mode ultrasound image.
Fig. 5 shows an exemplary pulsed wave Doppler ultrasound image.
Fig. 6A shows an exemplary flow contour in an artery.
Fig. 6B shows an exemplary flow profile in an artery.
Fig. 7 shows an exemplary arterial branch.

Fig. 8 shows an exemplary color Doppler ultrasound image.
Fig. 9 shows an exemplary relationship between the maximum velocity shift and the Dean number.
Fig. 10 shows an exemplary flow profile.

## DESCRIPTION OF EMBODIMENTS

The invention will be described herein with reference to the figures.

It should be understood that the following description, while indicating exemplary embodiments, is intended for purposes of illustration only, and not intended to limit the scope of the invention. It should also be understood that the Figs are mere schematics and not drawn to scale. It should also be understood that the same reference numerals are used throughout the Figs to indicate the same or similar parts.

The invention provides a computer-implemented method and system to carry out said computer-implemented method, to determine a flow profile within an artery from ultrasound imaging data. The flow profile may be a 2D flow profile and/or a 3D flow profile. According to an exemplary embodiment, the computer-implemented method may also be used to determine a curvature of an artery. As disclosed herein, the method may be carried out by a system during image acquisition, i.e., in real time, or may be carried out *a posteriori* in a subsequent step, at any time after image acquisition.

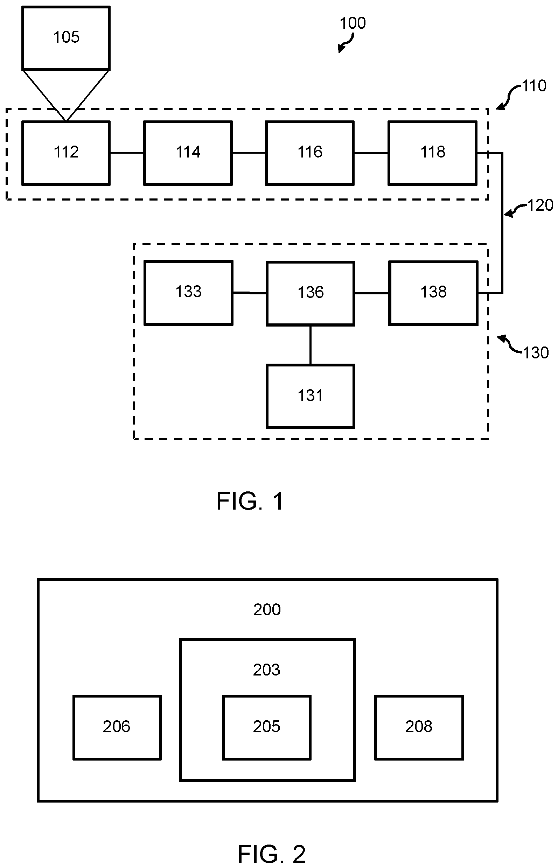

Fig. 1 shows a schematic diagram of an exemplary ultrasound imaging system 100. The system 100 includes an ultrasound imaging probe 110 in communication with a host 130 over a communication interface or link 120. The probe 110 may include a transducer array 112, a beamformer 114, a processor 116, and a communication interface 118. The host 130 may include a display 131, a processor 136, a communication interface 138, and a memory 133. The host 130 and/or the processor 136 of the host 130 may also be in communication with other types of systems or devices in replacement or addition to the here mentioned systems such as for example, an external memory, external display, a subject tracking system, an inertial measurement unit, etc. It is understood that the beamformer may also be a microbeamformer. It is further understood that the components as shown here may also be configured in alternate arrangements. For example, the processor 116 and/or the beamformer 114 may be located outside of the probe 110 and/or the display 131 and/or memory 133 may be located outside of the host 130.

In some embodiments, the probe 110 is an external ultrasound imaging device including a housing configured for handheld operation by a user. The transducer array 112 can be configured to obtain ultrasound data while the user grasps the housing of the probe 110 such that the transducer array 112 is positioned adjacent to or in contact with a patient's skin. The probe 110 is configured to obtain ultrasound data of anatomy within the patient's body while the probe 110 is positioned outside of the patient's body. In some embodiments, the probe 110 can be a patch-based external ultrasound probe. For example, the probe may be a hemodynamic patch.

In other embodiments, the probe 110 can be an internal ultrasound imaging device and may comprise a housing configured to be positioned within a patient's body, including the patient's coronary vasculature, peripheral vasculature, esophagus, heart chamber, or other body or body cavity. In some embodiments, the probe 110 may be an intravascular ultrasound (IVUS) imaging catheter or an intracardiac echocardiography (ICE) catheter. In other embodiments, probe 110 may be a transesophageal echocardiography (TEE) probe. Probe 110 may be of any suitable form for any suitable ultrasound imaging application including both external and internal ultrasound imaging.

The transducer array 112 emits ultrasound signals towards an anatomical object 105 of a patient and receives echo signals reflected from the object 105 back to the transducer array 112. The transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a patient's anatomy. In some embodiments, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The beamformer 114 is coupled to the transducer array 112. The beamformer 114 controls the transducer array 112, for example, for transmission of the ultrasound signals and reception of the ultrasound echo signals. In some embodiments, the beamformer 114 may apply a time-delay to signals sent to individual acoustic transducers within an array in the transducer array 112 such that an acoustic signal is steered in any suitable direction propagating away from the probe 110. The beamformer 114 may further provide image signals to the processor 116 based on the response of the received ultrasound echo signals. The beamformer 114 may include multiple stages of beamforming. The beamforming can reduce the number of signal lines for coupling to the processor 116. In some embodiments, the transducer array 112 in combination with the beamformer 114 may be referred to as an ultrasound imaging component. The beamformer 114 may also be a microbeamformer.

The processor 116 is coupled to the beamformer 114. The processor 116 may also be described as a processor circuit, which can include other components in communication with the processor 116, such as a memory, beamformer 114, communication interface 118, and/or other suitable components. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 Wi-Fi link, or a Bluetooth link.

At the host 130, the communication interface 138 may receive the image signals. The communication interface 138 may be substantially similar to the communication interface 118. The host 130 may be any suitable computing and display device, such as a workstation, a personal computer (PC), a laptop, a tablet, or a mobile phone.

The processor 136 is coupled to the communication interface 138. The processor 136 may also be described as a processor circuit, which can include other components in communication with the processor 136, such as the memory 133, the communication interface 138, and/or other suitable components. The processor 136 can be configured to generate image data from the image signals received from the probe 110. The processor 136 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some embodiments, the processor 136 can form a three-dimensional (3D) volume image from the image data. In some embodiments, the processor 136 can perform real-time processing on the image data to provide a streaming video of ultrasound images of the object 105.

The memory 133 is coupled to the processor 136. The memory 133 can be configured to store patient information, measurements, data, or files relating to a patient's medical history, history of procedures performed, anatomical or biological features, characteristics, or medical conditions associated with a patient, computer readable instructions, such as code, software, or other application, as well as any other suitable information or data. The memory 133 may be located within the host 130. There may also be an additional external memory, or an external memory in replacement of memory 133. An external memory may be a cloud-based server or an external storage device, located outside of the host 130 and in communication with the host 130 and/or processor 136 of the host via a suitable communication link as disclosed with reference to communication link 120. Patient information may include measurements, data, files, other forms of medical history, such as but not limited to ultrasound images, ultrasound videos, and/or any imaging information relating to the patient's anatomy. The patient information may include parameters related to an imaging procedure such a probe position and/or orientation.

The display 131 is coupled to the processor 136. The display 131 may be a monitor or any suitable display or display device. The display 131 is configured to display the ultrasound images, image videos, and/or any imaging information of the object 105.

The system 100 may be used to assist a sonographer or operator in performing an ultrasound scan. The scan may be performed in a point-of-care setting. In some instances, the host 130 is a console or movable cart. In some instances, the host 130 may be a mobile device, such as a tablet, a mobile phone, or portable computer. In yet other examples the host is a server on a cloud and an external display connects to the host in the cloud. During an imaging procedure, the ultrasound system 100 can acquire an ultrasound image of a region of interest of a subject.

In particular, the ultrasound system 100 may acquire ultrasound images of an artery. More specifically, the ultrasound system 100 may be configured to acquire ultrasound imaging data in accordance with method 300 discussed below including ultrasound data from B-mode ultrasound, Pulsed Wave Doppler mode ultrasound and color or power Doppler mode ultrasound. These three ultrasound modes may be obtained from a single instruction in a "so-called" triplex ultrasound mode, in which three ultrasound modes are used simultaneously to acquire ultrasound data in each of the three modes. In a preferred *modus operandi,* the ultrasound probe will be placed approximately perpendicular, and at an angle of approximately 50 to 70 degrees with respect to the artery, to acquire ultrasound data. It is noted that other angles, between 1 and 89 degrees as also envisaged by the present disclosure.

Fig. 2 is a schematic diagram of a processor circuit. The processor circuit 200 may be implemented in the probe 110 and/or the host system 130 of Fig. 1, or any other suitable location, such as an external computing system separate from an image acquisition devise or system. One or more processor circuits can be configured to carry out the operations described herein. The processor circuit 200 can be part of the processor 116 and/or processor 136 or may be separate circuit. In an example, the processor circuit 200 may be in communication with the transducer array 112, beamformer 114, communication interface 118, communication interface 138, memory 133 and/or the display 131, as well as any other suitable component or circuit within ultrasound system 100. As shown, the processor circuit 200 may include a processor 206, a memory 203, and a communication module 208. These elements may be in direct or indirect communication with each other, for example via one or more buses. The processor circuit may further be in communication with an external storage and/or to an external display. The external storage may be used to retrieve or store data, including images, ultrasound data and/or patient information. The external display may be used to display outputs and/or to control the processing circuit, for example by giving instructions.

A processor 116, 136, 206 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. A processor 116, 136, 206 may also be implemented as a combination of computing devices, e.g., a combination of a DSP and a microprocessor, a plurality of microprocessors, one or more microprocessors in conjunction with a DSP core, or any other such configuration. The processor 260 may also implement various deep learning networks, which may include a hardware or a software implementation. The processor 206 may additionally include a preprocessor in either hardware or software implementation.

A memory 118, 138, 208 as envisaged by the present disclosure may be any suitable storage device, such as a cache memory (e.g., a cache memory of the processor 116, 136, 206), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processing circuit. In an embodiment, the memory 203 may store instructions 205. The instructions 205 may include instructions that, when executed by a processor 116, 136, 206, cause the processor 116, 136, 206 to perform the operations described herein with reference to the probe 110 and/or the host 130 (Fig. 1).

Instructions 205 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 205 may be in the form of an executable computer program or script. For example, routines, subroutines and/or functions may be defined in a programming language including but not limited to C, C++, C#, Pascal, BASIC, API calls, HTML, XHTML, XML, ASP scripts, JavaScript, FORTRAN, COBOL, Perl, Java, ADA, .NET, and the like. In particular, according to the present disclosure, instructions as disclosed with reference to method 300 are envisaged herein.

The communication module 208 can include any electronic circuitry and/or logic circuitry to facilitate direct or indirect communication of data between the processor circuit 200, the probe 110, the host 130, and/or the display 131. In that regard, the communication module 208 can be an input/output (I/O) device. In some instances, the communication module 208 facilitates direct or indirect communication between various elements of the processor circuit 200, the probe 110 (Fig. 1) and/or the host 130 (Fig. 1).

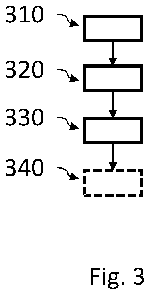

According to an exemplary embodiment, the computer-implemented method contains the steps as disclosed in Fig. 3.

In step 310, ultrasound imaging data of an artery is received. The ultrasound data may be received in real time, i.e., during image acquisition, or may be queried upon request from a user or machine from a storage facility, for example, a storage or memory within the image acquisition device, a cloud, or a physical hard drive external to the image acquisition device. According to the invention, the ultrasound data comprises any combination including:

- B-mode ultrasound data,
- pulsed wave Doppler ultrasound data, and
- color or power Doppler ultrasound data.

It is understood by a person skilled in the art that other forms of ultrasound data, in addition and/or in replacement of the above ultrasound data may also be received.

According to an embodiment of the invention, the ultrasound data may be received from an ultrasound device or system as provided in Fig. 1 comprising an array of acoustic elements 112 configured to acquire

- B-mode ultrasound data,
- pulsed wave Doppler ultrasound data, and
- color or power Doppler ultrasound data.

The acquisition mode may be pre-programmed, e.g., as a triplex ultrasound imaging mode, in such a manner that the

- B-mode ultrasound data,
- pulsed wave Doppler ultrasound data, and
- color or power Doppler ultrasound data

are obtained from a sequential or alternating ultrasound shots. This pre-programmed control may be executed upon a user request to use said triplex mode, or upon a machine command in case a velocity profile is selected to be determined.

According to an embodiment, the ultrasound device comprising an acoustic array configured for triplex mode as described above, may be a hemodynamic 1D ultrasound patch, e.g., an ultrasound patch that may be attached on a subject's skin for continued monitoring of a feature within the subject (e.g., heart, lungs, etc.). In such a case, the array of acoustic elements may acquire data continuously in triplex mode (e.g., by acquiring ultrasound data simultaneously in each mode or by alternating the different modes within the triplex mode). In other words, acquire data of sufficient time length in for example, first B-mode, then pulsed wave Doppler mode and last in color or power Doppler mode, and then repeat acquisition. It should be understood that any order of these modes is envisaged by the invention.

In step 320, the ultrasound data is processed in order to determine a first set of properties or measurements from the artery. In particular, in step 320

- a dimension of an artery,
- a velocity in an artery and
- a measure of flow profile asymmetry

are determined from the ultrasound data.

An artery dimension (or lumen dimension) refers to any dimension relating to a cross-section of the artery. An artery dimension (or lumen dimension) may thus, for example, be the artery diameter or radius. The artery dimension may however also be for example the surface area or perimeter of a transversal cross-section of the artery. The artery or lumen dimension may be obtained from for example B-mode ultrasound data.

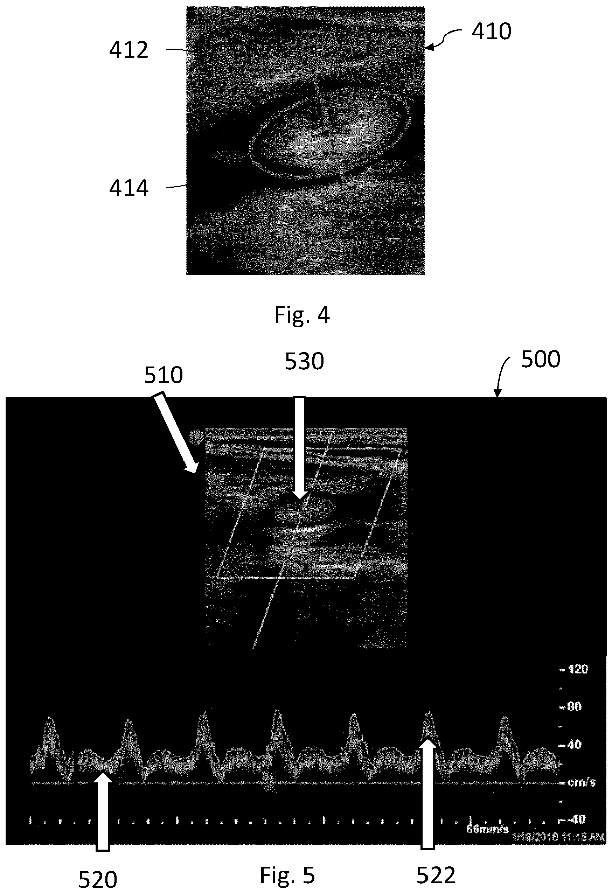

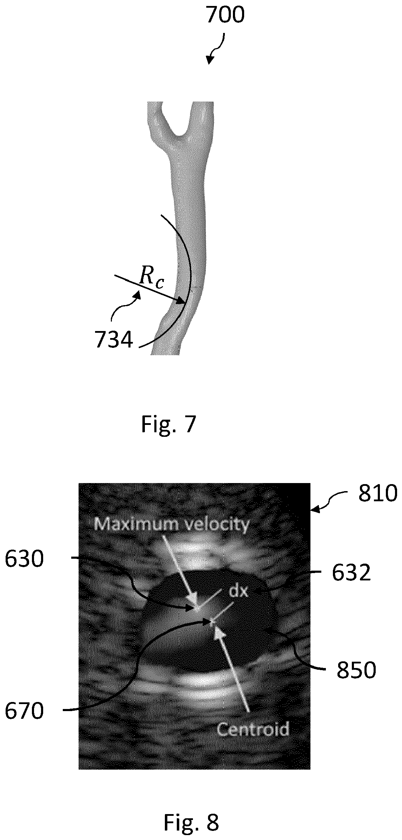

Fig. 4 displays an ultrasound image 410 generated from B-mode ultrasound data of a cross-section of an artery according to an embodiment of the invention. In particular, Fig. 4 displays a cross-section of an artery at approximately 20 to 40 degrees with respect to the length of the artery and approximately 50 to 70 degrees with respect to a transversal cross-section, such that the diameter or radius may be obtained from the minor axes 412 of the illustrative oval 414 drawn merely for illustrative purposes. It should be clear to a person skilled in the art, that other ultrasound data may also be suitable to determine a dimension of the artery, such as for example color or power Doppler ultrasound data shown in Fig. 8. It should also be clear to a person skilled in the art, that artery dimension and lumen dimension in the context of this invention may be used interchangeably.

According to an embodiment of the invention, a velocity in the artery refers to a local velocity as measured in an area smaller than the area of a transversal artery cross-section. A velocity in the artery is thus a local velocity at any given location or area within the lumen of the artery. For example, a velocity in an artery may be the velocity in the center of the artery. According to another example, a velocity may be the velocity at a pre-defined location within the artery, such as for example the location of the maximum velocity within the artery, or a location near an edge of the artery. A velocity in the artery may be determined from any ultrasound mode, such as color Doppler, power Doppler or pulsed wave Doppler ultrasound. In an embodiment, a velocity is determined from pulsed wave Doppler ultrasound data. The pulsed wave Doppler ultrasound mode, for example, allows to measure a local velocity at a higher accuracy than in color or power Doppler ultrasound mode. Due to the high frame rate, pulsed wave Doppler ultrasound mode, may thus also be advantageous to resolve important features of the velocity waveform, e.g., the systolic peak velocities, in an artery.

Fig. 5 shows visualized ultrasound data 500 obtained from a pulsed wave Doppler ultrasound mode. The ultrasound data includes an ultrasound image 510 and a timeline of a velocity 520, wherein the velocity is determined within a relatively small sampling region 530. The timeline displays an evolution of the velocity within the sampling area 530 over time, resolving, due to the high frame rate of pulsed wave Doppler, systolic peaks 522. A velocity at a given location within the artery or lumen, may thus be determined as an instantaneous velocity, e.g., a single point from the timeline 520, or as an average velocity, averaging over the velocity in the timeline 520. Additionally, the sampling region 530, may be as shown in Fig. 5, at the center of the artery, or at any point within the cross-section, such as for example the maximum velocity location determined from color Doppler-mode ultrasound data.

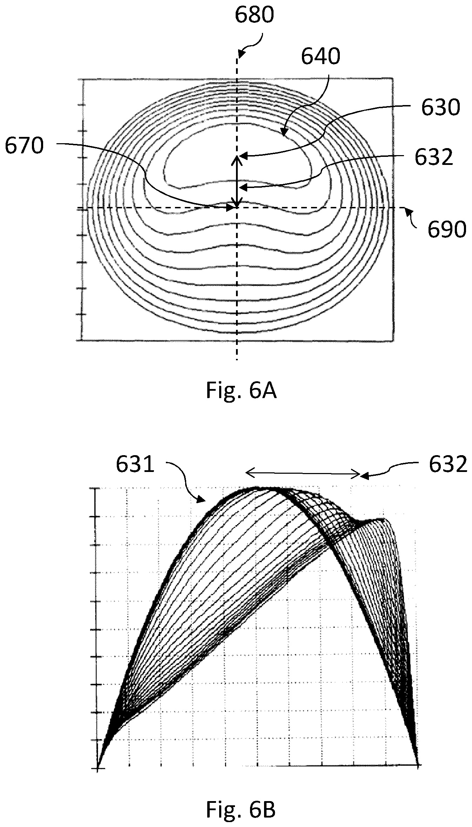

According to an embodiment of the invention, a measure of asymmetry of the flow refers to any parameter which indicates a level of asymmetry or skewedness in a flow profile with respect to a symmetric flow profile. For example, a measure of asymmetry may be a deviation of a flow profile from a symmetric parabolic flow profile. In another example, a measure of asymmetry may be a deviation of the velocity maximum from the center axes of the artery or centroid of an artery cross-section. For example, in curved arteries, so-called Dean vortices may arise and skew the typically symmetric parabolic flow profile observed in straight arteries, in such a way that the maximum velocity within the artery may no longer lie at the central axes of the artery, but at a certain distance, shifted away from the central axes of the artery. Such deviations of the flow are shown in Figs. 6A and 6B.

Fig. 6A displays a 2D velocity distribution in a curved vessel as a contour plot. Each line in the contour plot represents a particular velocity. For example, starting at a zero velocity for the most outer line, each next line towards the center of the Fig represents an increase velocity.

In a typically straight artery, the velocity profile may be entirely symmetric across both shown axes 680 and 690 (these axes may be at any orientation, as long as the two axes are perpendicular to each other). In curved arteries however, due to the Dean vortices, the velocity distribution may shift to be asymmetric across at least one axes 690. The maximum velocity, being in the center of the area delimited by the most inner contour line 640, thus shifts from the centroid 670 by a distance 632 to a new location 630. The distance that the velocity maximum shifts is determined by the radius of curvature $R_c$ 734 of the artery 700 as shown in Fig. 7.

Fig. 6B displays a velocity profile along the asymmetric axis 680 for various radii of curvature 734. For example, an artery with a zero radius of curvature would display a fully symmetric flow profile 631. As the radius of curvature increases the velocity maximum shifts by a distance 632, wherein the distance 632 increases as the radius of curvature increases.

Fig. 8 displays an ultrasound image 810 from color or power Doppler ultrasound data with a heat map 850 of the velocity in a cross-section of the artery. While the heat map may be qualitative, e.g., without any indication of absolute velocity values, it may provide information with regards to the velocity maximum 630 due to a change in color, color intensity and/or brightness at the maximum. The shift 632 of the velocity maximum 630 with respect to the centroid 670 of the artery cross-section may then be determined by, for example, calculating a distance 632 between the velocity maximum location 630 and the centroid 670 of the artery cross-section.

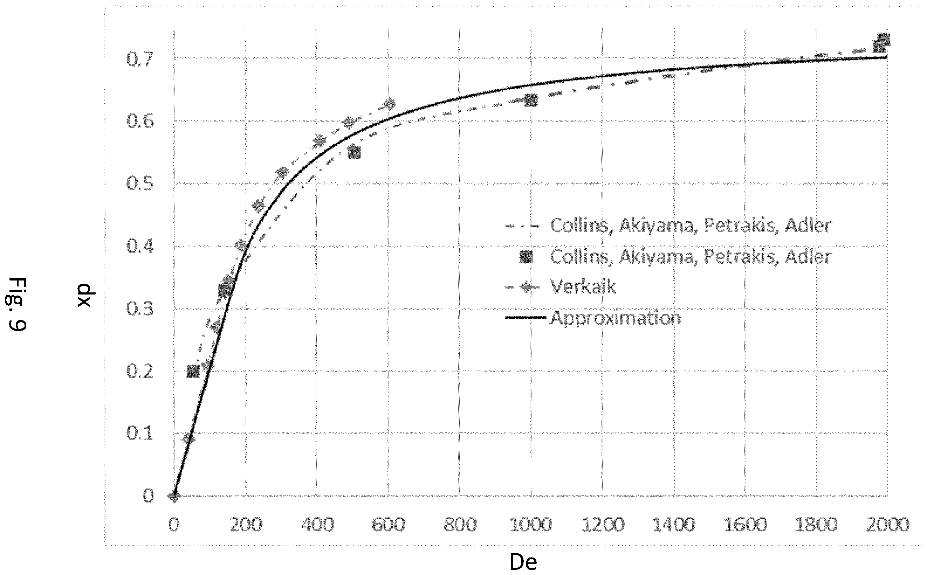

According to an embodiment of the invention, the velocity maximum shift 632 may be associated to a non-dimensional parameter such as a local Dean number $De$. In particular, it has been shown for at least self-similar flows in, for example, Cieslicki et al. (2012) "Can the Dean number alone characterize flow similarity in differently bent tubes?", Verkaik et al. (2009) "Estimation of volume flow in curved tubes based on analytical and computational analysis of axial velocity profiles", and Petrakis et al. (2009) "Steady Flow in a Curved Pipe with Circular Cross-Section. Comparison of Numerical and Experimental Results", that the shift $dx$ 632 of the velocity maximum location is dependent on the Dean number De according to Fig. 9. In other words, the local Dean number may be extracted from for example Fig. 9 by merely having determined the shift 632 of the maximum velocity, without requiring a value for the velocity. For example, the maximum velocity shift may be determined to be 40 % of the artery diameter, such that the local Dean number corresponds to approximately 200.

It is noted that the present invention also envisages approaches in which the Dean number as obtained according to the velocity shift and Fig. 9 may only be a first estimation and may be recalculated at a later stage. The Dean number may for example be determined iteratively to converge to a value by combining blocks of the method 300 in a different order, and/or adding and/or removing blocks of the method 300.

With reference again to Fig. 3, in step 330, a flow profile within the artery is determined. For example, the color or power Doppler ultrasound data, may be integrated pixel by pixel over the heat map 850 of Fig. 8. In another example, a flow profile is determined based on the first set of properties as determined in step 320 in combination with an analytic expression. For example, an artery dimension, a velocity in an artery and a measure of asymmetry may be combined to determine a flow profile. In the following various suitable methods will be presented to determine a flow profile.

According to an embodiment based on Soedberg (1987) "Viscous flow in curved tubes - I. velocity profiles", a non-dimensional or normalized flow profile u(r), passing through the maximum velocity, may be determined from an artery dimension and a measure of asymmetry following

$$u(r) = w_0(r) - w_1(r) + \cdots \qquad (1)$$

were two first terms are given by

$$w_0(r) = \frac{(1 - r^{F-1})F}{(F-1)},$$

and

$$w_1(r) = 1 - 0.3r - 0.7r^{\sqrt{q-0.95}}.$$

Here $w_0$ and $w_1$ represent the zeroth and first tangential harmonic contributions to the flow. Higher harmonic contributions may also be considered, such as for example the second, third and so on harmonic contributions. In addition or in replacement, also different expressions of for the harmonic contributions may be used such as

$$w(r) = 2\frac{(1 - r^{F-1})F}{(F-1)},$$

Further, r represents a radial coordinate normalized by the total artery radius R (i.e., r= r'/R), which may be derived as described above. It should be noted, a velocity profile along any radial direction of a cross-section may be obtained. For example, one velocity profile u(r) may be along the axis 680 in Fig. 6A, and another velocity profile u(r) may be along the axis 690 shown in Fig. 6A.
Further, F represents a form factor which may be any one of:

$$F = 0.5 \cdot q^{0.6} + 1.5,$$

$$F = 0.5(1 + q^{0.5}) + 1.5,$$

$$F = 0.5(1 + q)^{0.6} + 1.5,$$

with

$$q = \text{De}/16,$$

where De is the local Dean number, representative of a measure of asymmetry, and which may be derived from the shift of the maximum velocity as discussed above under step 320 of the method 300.
According to an embodiment based on Tijssen (1979) "Axial dispersion in helically coiled open columns for chromatography", the flow profile may be estimated according to

$$u(r) = q^2(1-r)^2 \exp\{(1-r)(\ln(8/9) - 0.5\,\ln[(q/1.6)^{3.4} + 1])\} \tag{2}$$

or

$$u(r) = \frac{F^2}{F-1}(1 - r^F)\exp\left[(1 - r^2)(1 - a\,\ln F^b)\right] \tag{3}$$

where in addition to the variable definitions above *a* is typically between 0.2 and 0.3, preferably 0.25 and *b* is between 3.5 and 4, preferably 3.75. It should be understood that *a* and *b* are fitting constants, which may thus deviate from the mentioned values, may be removed or replaced by different fitting constants.
Following the above expressions for u(r), the obtained velocity profile may be accurate in shape, but not in absolute values, given that it concerns a normalized velocity profile. Therefore, the normalized velocity profile may be shifted or scaled by a velocity in an artery, for example, by a velocity, derived from a pulsed wave Doppler measurement. In such a case, the normalized velocity profile u(r), which may be any velocity profile as shown in Fig. 6B, may be shifted from non-dimensional normalized units to having units of for example distance over time (i.e., m/s, cm/s, feet/s, etc.).

The inventors have further appreciated that the accuracy of the velocity distribution obtained following Eq. (1) may be within 10 % of the real velocity distribution when 16 < De < 240, and that the accuracy of the velocity distribution obtained following Eqs. (2) or (3) may be within 10 % of the real velocity distribution when De > 100.

According to an embodiment, Eq. (1) and either of Eqs. (2) or (3) may thus be combined, such that for lower *De* numbers Eq. (1) is used and for higher *De* numbers either of Eqs. (2) or (3) may be used in order to obtain an accuracy of the velocity profile within 10 % for a greater De number range. The relevant *De* range within an artery may for example be 1 < De < 2000.

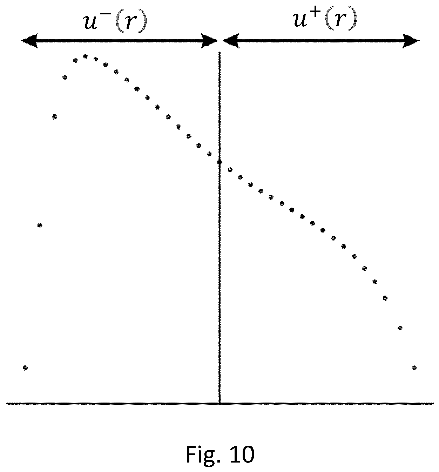

According to an embodiment, the flow profile (this may be the non-dimensional normalized flow profile or the scaled and dimensional flow profile) may further be used to determine a 3D flow profile as proposed in Verkaik et al. (2009). The cross section (e.g., as shown in Fig. 6A) is divided into two semicircles along the diameter perpendicular to that on which the flow profile is known. The flow rate $Q(r, \theta)$ is then estimated by assuming the axial flow to be axisymmetric in each semicircle as shown in Fig. 10, giving the expression

$$Q(r, \theta) = \pi \int_0^R u^+(r) r\, dr + \pi \int_0^R u^-(r) r\, dr.$$

This expression may be solved according to integration by parts.

In an optional step 340, a radius of curvature $R_c$ 734 may be determined from the flow profile $u(r)$.

i) From the flow profile determined in step 130, a Reynolds number Re may be determined following

$$Re = \frac{\rho U 2R}{\mu}.$$

Here $\rho$ is the blood density, $U$ is the average velocity in the artery at the imaged cross-section, $D$ and $\mu$ is the dynamic viscosity. The average velocity may be obtained as:

- half the maximum velocity for laminar flows,
- the center velocity for turbulent flows, or
- as the average velocity of the entire cross-section, which may be obtained through mathematical averaging.

ii) The radius of curvature may be obtained relating the Reynolds number to the Dean number according to

$$De = Re \sqrt{\frac{2R}{2R_c}}.$$

It is understood by a person skilled in the art that while method 300 was presented in a specific order, the order of the steps may vary, additional steps may be added in between, and/or steps may be removed.

It is furthermore understood that the individual steps in the method 300 may be performed in real time, i.e., during an examination procedure, or thereafter. For example, the ultrasound data may be obtained in real time and analyzed according to method 300 during an image acquisition procedure, or the imaging data may be first obtained during an acquisition step and only be analyzed upon request of an operator and/or expert. The method 100 may thus be implemented within an image acquisition system (Fig. 1) or a hemodynamic patch or outside of it in a separate computing unit (Fig. 2). In another embodiment the method 100 may also be carried out in a cloud.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

**Claims**

1. A computer-implemented method (300) for determining a flow profile in an artery based on ultrasound imaging data, the method comprising:

    - receiving (310) ultrasound imaging data of an artery in triplex mode, wherein the triplex mode comprises:

        - B-mode ultrasound data,
        - pulsed wave Doppler ultrasound data, and
        - color or power Doppler ultrasound data;

    - determining (320), from the ultrasound imaging data,

        - an artery dimension;
        - a velocity in the artery cross-section, and
        - a measure of asymmetry of the flow profile; and

    - determining (330) a flow profile from

        - the artery dimension,
        - the velocity in the artery cross-section, and
        - the measure of asymmetry of the flow profile.

2. The method of any of the preceding claims further comprising:

    - determining (340) a local artery curvature based on

        - the artery dimension,
        - the flow profile, and
        - the measure of asymmetry of the flow profile.

3. The method of any of the preceding claims, wherein the artery dimension is determined based on the B-mode ultrasound data.

4. The method of any of the preceding claims, wherein the velocity in the artery cross-section is determined based on the pulsed wave Doppler ultrasound data.

5. The method of any of the preceding claims, wherein the velocity in the artery cross-section is determined in a center of the artery cross-section.

6. The method of any of the preceding claims, wherein the measure of asymmetry of the flow profile is determined based on the color or power Doppler ultrasound data.

7. The method of any of the preceding claims, wherein the measure of asymmetry comprises:

    - determining a location of the maximum flow velocity in an artery cross-section; and based on said location, determining a parameter representative of a deviation of said location with respect to the center of the artery cross-section.

8. The method of claim 7, wherein the parameter representative of the deviation of the location of the maximum flow velocity in an artery cross-section is the Dean number.

9. A computer program product comprising instructions for enabling a processor to carry out the method of any of the preceding claims.

10. A system (100) for determining a flow profile from ultrasound imaging data, the system comprising a processor configured to carry out the method of any of the claims 1-8.

11. The system of claim 10, further comprising:

- an array of acoustic elements (112) in communication with the processor (116, 136), and
- wherein the processor is configured to control the array of acoustic elements to send and receive acoustic signals to generate ultrasound imaging data.

12. The system of claim 10 or 11, wherein the system is a hemodynamic monitoring patch.


**Patentansprüche**

1. Computerimplementiertes Verfahren (300) zum Bestimmen eines Strömungsprofils in einer Arterie basierend auf Ultraschallbildgebungsdaten, wobei das Verfahren umfasst:

- Empfangen (310) von Ultraschallbildgebungsdaten einer Arterie im Triplexmodus, wobei der Triplexmodus umfasst:

- B-Mode-Ultraschalldaten,
- Doppler-Ultraschalldaten mit gepulsten Wellen, und
- Farb- oder Leistungs-Doppler-Ultraschalldaten; Bestimmen (320) aus den Ultraschallbildgebungsdaten
- einer Arteriendimension;
- einer Geschwindigkeit im Arterienquerschnitt, und
- eines Asymmetriemaßes des Strömungsprofils; und Bestimmen (330) eines Strömungsprofils aus
- der Arteriendimension,
- der Geschwindigkeit im Arterienquerschnitt, und
- dem Asymmetriemaß des Strömungsprofils.

2. Verfahren nach einem der vorstehenden Ansprüche, weiter umfassend:

- Bestimmen (340) einer lokalen Arterienkrümmung basierend auf

- der Arteriendimension,
- dem Strömungsprofil, und
- dem Asymmetriemaß des Strömungsprofils.

3. Verfahren nach einem der vorstehenden Ansprüche, wobei die Arteriendimension basierend auf den B-Mode-Ultraschalldaten bestimmt wird.

4. Verfahren nach einem der vorstehenden Ansprüche, wobei die Geschwindigkeit im Arterienquerschnitt basierend auf den Doppler-Ultraschalldaten mit gepulsten Wellen bestimmt wird.

5. Verfahren nach einem der vorstehenden Ansprüche, wobei die Geschwindigkeit im Arterienquerschnitt in einer Mitte des Arterienquerschnitts bestimmt wird.

6. Verfahren nach einem der vorstehenden Ansprüche, wobei das Asymmetriemaß des Strömungsprofils basierend auf den Farb- oder Leistungs-Doppler-Ultraschalldaten bestimmt wird.

7. Verfahren nach einem der vorstehenden Ansprüche, wobei das Asymmetriemaß umfasst:

- Bestimmen einer Stelle der maximalen Strömungsgeschwindigkeit in einem Arterienquerschnitt; und, basierend auf der Stelle, Bestimmen eines Parameters, der repräsentativ für eine Abweichung der Stelle in Bezug auf die Mitte des Arterienquerschnitts ist.

8. Verfahren nach Anspruch 7, wobei der Parameter, der repräsentativ für die Abweichung der Stelle der maximalen Strömungsgeschwindigkeit in einem Arterienquerschnitt ist, die Dean-Zahl ist.

9. Computerprogrammprodukt, das Anweisungen umfasst, um es einem Prozessor zu ermöglichen, das Verfahren nach einem der vorstehenden Ansprüche auszuführen.

10. System (100) zum Bestimmen eines Strömungsprofils aus Ultraschallbildgebungsdaten, wobei das System einen Prozessor umfasst, der dazu konfiguriert ist, das Verfahren nach einem der Ansprüche 1-8 auszuführen.

11. System nach Anspruch 10, weiter umfassend:

   - eine Anordnung akustischer Elemente (112) in Kommunikation mit dem Prozessor (116, 136), und
   - wobei der Prozessor dazu konfiguriert ist, die Anordnung akustischer Elemente zu steuern, um akustische Signale zu senden und zu empfangen, um Ultraschallbildgebungsdaten zu erzeugen.

12. System nach Anspruch 10 oder 11, wobei das System ein Pflaster zur hämodynamischen Überwachung ist.


**Revendications**

1. Procédé mis en oeuvre par ordinateur (300) pour déterminer un profil d'écoulement dans une artère sur la base de données d'imagerie par ultrasons, le procédé comprenant :

   - la réception (310) de données d'imagerie par ultrasons d'une artère en mode triplex, dans lequel le mode triplex comprend :

      - des données d'ultrasons en mode B,
      - des données d'ultrasons Doppler à ondes pulsées, et
      - des données d'ultrasons Doppler de couleur ou de puissance ;

   - la détermination (320), à partir des données d'imagerie par ultrasons,

      - d'une dimension d'artère ;
      - d'une vitesse dans la section transversale de l'artère, et
      - d'une mesure d'asymétrie du profil d'écoulement ; et

   - la détermination (330) d'un profil d'écoulement à partir de

      - la dimension d'artère,
      - la vitesse dans la section transversale de l'artère, et
      - la mesure d'asymétrie du profil d'écoulement.

2. Procédé selon l'une quelconque des revendications précédentes comprenant en outre :

   - la détermination (340) d'une courbure d'artère locale sur la base de

      - la dimension d'artère,
      - le profil d'écoulement, et
      - la mesure d'asymétrie du profil d'écoulement.

3. Procédé selon l'une quelconque des revendications précédentes, dans lequel la dimension d'artère est déterminée sur la base des données d'ultrasons en mode B.

4. Procédé selon l'une quelconque des revendications précédentes, dans lequel la vitesse dans la section transversale de l'artère est déterminée sur la base des données d'ultrasons Doppler à ondes pulsées.

5. Procédé selon l'une quelconque des revendications précédentes, dans lequel la vitesse dans la section transversale de l'artère est déterminée dans un centre de la section transversale de l'artère.

6. Procédé selon l'une quelconque des revendications précédentes, dans lequel la mesure d'asymétrie du profil d'écoulement est déterminée sur la base des données d'ultrasons Doppler de couleur ou de puissance.

7. Procédé selon l'une quelconque des revendications précédentes, dans lequel la mesure d'asymétrie comprend :

   - la détermination d'un emplacement de la vitesse d'écoulement maximale dans une section transversale de l'artère ; et, sur la base dudit emplacement, la détermination d'un paramètre représentatif d'un écart dudit emplacement par rapport au centre de la section transversale de l'artère.

8. Procédé selon la revendication 7, dans lequel le paramètre représentatif de l'écart de l'emplacement de la vitesse d'écoulement maximale dans une section transversale de l'artère est le nombre de Dean.

9. Produit de programme informatique comprenant des instructions pour permettre à un processeur de mettre en oeuvre le procédé selon l'une quelconque des revendications précédentes.

10. Système (100) pour déterminer un profil d'écoulement à partir de données d'imagerie par ultrasons, le système comprenant un processeur configuré pour mettre en oeuvre le procédé selon l'une quelconque des revendications 1-8.

11. Système selon la revendication 10, comprenant en outre :

   - un réseau d'éléments acoustiques (112) en communication avec le processeur (116, 136), et
   - dans lequel le processeur est configuré pour commander le réseau d'éléments acoustiques pour envoyer et recevoir des signaux acoustiques pour générer des données d'imagerie par ultrasons.

12. Système selon la revendication 10 ou 11, dans lequel le système est un patch de surveillance hémodynamique.

FIG. 1

FIG. 2

310

320

330

340

Fig. 3

412

410

414

Fig. 4

510

530

500

520

Fig. 5

522

Fig. 6A

Fig. 6B

700

$R_c$

734

Fig. 7

810

Maximum velocity

630

632

dx

670

850

Centroid

Fig. 8

Fig. 9

Fig. 10

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120078106 A

**Non-patent literature cited in the description**

- **VERKAIK et al.** *Estimation of volume flow in curved tubes based on analytical and computational analysis of axial velocity profiles,* 2009
- **PETRAKIS et al.** *Steady Flow in a Curved Pipe with Circular Cross-Section. Comparison of Numerical and Experimental Results,* 2009
- **SOEDBERG.** *Viscous flow in curved tubes - I. velocity profiles,* 1987
- **TIJSSEN.** *Axial dispersion in helically coiled open columns for chromatography,* 1979